# EUROPEAN PATENT SPECIFICATION

(11) **EP 1 788 077 B1**
(45) Date of publication and mention of the grant of the patent: **14.09.2016**
(21) Application number: 06255896.0
(22) Date of filing: 17.11.2006
(51) Int. Cl.: C12N 5/00, A61L 27/00, C12N 5/071

(54) **A method for creating cell clusters**
Methode zur Herstellung von Zellmassen
Procédé de générer des groupes de cellules

(30) Priority: 18.11.2005 US 738171 P
(43) Date of publication of application: 23.05.2007
(60) Divisional application: 10179799.1
(73) Proprietor: LifeScan, Inc., Milpitas, CA 95035 (US)
(72) Inventor: Ghabrial, Ragae M., Helmetta, NJ 08828 (US)
(74) Representative: Goodfellow, Hugh Robin

(56) References cited:
- EP-A1- 1 939 280
- KOIDE N ET AL: "FORMATION OF MULTICELLULAR SPHEROIDS COMPOSED OF ADULT RAT HEPATOCYTES IN DISHES WITH POSITIVELY CHARGED SURFACES AND UNDER OTHER NONADHERENT ENVIRONMENTS" EXPERIMENTAL CELL RESEARCH, SAN DIEGO, CA, US, vol. 186, 1990, pages 227-235, XP002938949 ISSN: 0014-4827
- KONNO ET AL: "Formation of embryoid bodies by mouse embryonic stem cells on plastic surfaces" JOURNAL OF BIOSCIENCE AND BIOENGINEERING, ELSEVIER, AMSTERDAM,, NL, vol. 100, no. 1, July 2005 (2005-07), pages 88-93, XP005665539 ISSN: 1389-1723
- BHATIA S.N. ET AL: "Controlling cell interactions by micropatterning in co-cultures: Hepatocytes and 3T3 fibroblasts", JOURNAL OF BIOMEDICAL MATERIALS RESEARCH, WILEY, NEW YORK, NY, US, vol. 34, no. 2, 1 January 1997 (1997-01-01), pages 189-199, XP002298019, ISSN: 0021-9304
- OTSUKA HIDENORI ET AL: "Two-dimensional multiarray formation of hepatocyte spheroids on a microfabricated PEG-brush surface", CHEMBIOCHEM - A EUROPEAN JOURNAL OF CHEMICAL BIOLOGY, WILEY VCH, WEINHEIM, DE, vol. 5, no. 6, 7 June 2004 (2004-06-07), pages 850-855, XP002298016, ISSN: 1439-4227

## Description

### FIELD OF THE INVENTION

This invention relates to *an in vitro* method for inducing cells to form three-dimensional cell clusters. It also further relates to a method for controlling the size and uniformity of such clusters.

### BACKGROUND

In mammalian *tissues in vivo,* cells exist in a complex three-dimensional (3-D) environment where cells are grown, either uniformly or non-uniformly, in three-dimensions around a central cell or group of cells. Cells are in close contact with neighboring cells, and are also connected to a mesh of extracellular matrix (ECM) that provides support for such cells. Cell fate decisions are governed by a complex interplay provided by cell-autonomous signals and stimuli from surrounding cells. It is well established that cell contact with ECM via integrins affects many cellular functions, such as proliferation, differentiation, migration, apoptosis, and cell shape. For example, in summarizing prior studies of pancreatic function, US Patent 6703017 reports a remarkable architecture and cellular organization of pancreatic islets that is ideal for rapid, yet finely controlled, responses to changes in blood glucose levels.

Riedl et al (Journal of Immunology, 2000, 165: 1381-1386) report that epithelial langerhans cell development from human CD34+ hemopoietic progenitor cells in response to TGF-β1 costimulation is associated with pronounced cell cluster formation of developing LC precursor cells. Epithelial langerhans cells form a three-dimensional network in suprabasal epidermal *layers in vivo* and stay in the epidermis for long periods of time, enabling them to fulfill a sentinel role in which they filter the surrounding tissue for foreign antigens.

However, in order to study these cellular processes *in vitro*, cells are typically isolated from tissues and grown in monolayers, which may be likened to a two-dimensional (2-D) culture systems where cells are grown in layers only one or two cells thick. This type of cell layer formation, however, alters inter-cellular interactions and the ECM and changes the complex interplay between integrins and cytoskeleton molecules.

Culturing cells in an *in vitro* environment that resembles the 3-D environment observed *in vivo* is extremely beneficial in allowing intimate contact in a manner similar to the original *in vivo* environment. In turn, this has a significant effect on cellular function. Embryonic stem cells (ESCs) clearly respond to the consequences of this phenomenon. When ESCs are allowed to differentiate in suspension culture, over time, they spontaneously form spherical multi-cellular aggregates or embryoid bodies (EB). It is thought that cells in an EB are involved in a high degree of communication and organization, which initiates their differentiation into all three germ layers.

Culturing cells on a 3-D solid support, such as, for example, a scaffold, is one approach to artificially restore the *in vivo* 3-D environment. However, cells initially continue to grow in a monolayer along the surface of the support, attaching preferentially to the substrate before they attach to each other.

Multi-cellular clusters have been widely used over the past four decades as *in vitro* systems to study, for example, mechanisms underlying organogenesis, responses of tumor cells to therapy, and interactions of epithelial-mesenchymal cells in tumors. These re-aggregated cell cultures were first introduced as an approach to understanding morphogenesis. A single cell suspension is obtained by enzymatic and/or mechanical dissociation and re-aggregated as 3-D clusters by a variety of methods. Tissue culture conditions are then provided such that the adhesive forces between cells are greater than those for the substrate on which the cells are plated. State of the art methods in developing clusters involves plating cells in gyratory shakers, roller flasks, or spinner flasks that continuously move and thus prevent cellular adherence to the vessel walls.

For example, US20040096967 outlines a method for the formation of EB by culturing cells in a suspension culture while applying a rotary shaking force. The disadvantage to this technique is the destructive sheer stress effect of the rotational force on the cells.

Cell clusters may also form spontaneously *in vitro*. US Patent 6,703,017 states Islet-like structures or islet progenitor cell-derived islets are highly organized structures of cells arising in culture indirectly from islet producing stem cells.

Another technique involves simply coating the tissue culture surfaces with a thin layer of agarose or other non-adhesive substance. Under these conditions, cells are not able to adhere to the surface of culture flasks; as a result, many cell types undergo homotypic aggregation.

For example, in a study to produce a cellular therapy for diabetes, Zayas et al. (EP 0 363 125, 1990), disclosed a process for proliferation of pancreatic endocrine cells. The process depends on the use of fetal pancreatic tissue, and a synthetic structure, including collagen, which is prepared to embed these cells for implantation.

Cells may also form clusters in response to the addition of a factor to the culture medium. For example, Riedl *et al* state "cytokine TGF-β1 plays a key role during epithelial langerhans cell development and differentiation. Using an *in vitro* differentiation model of CD34+ hemopoietic progenitor cells, we recently demonstrated that development of LC from CD34+ progenitor cells in a serum-free culture system is absolutely dependent on TGF-ß1 stimulation".

However, these techniques, although effective in facilitating cluster formation, have no means of controlling the size of the clusters or their uniformity. Once the clusters are formed in suspension, they continue increasing in size by adding other cells or by aggregating with neighboring clusters. This eventually reduces diffusion of oxygen and nutrients to the inner portion of the cluster and initiates a cascade of events leading to the death of cells in the cluster core.

### SUMMARY

The current invention comprises, among other things, *an in vitro* method for the controlled formation of a cell cluster. The clusters of the current invention may be formed of groups of single cells, clusters of cells, or combinations thereof. The cells comprising a cluster may be of a single type or several types. They may be stem cells or primary or expanded cells that are undifferentiated or partially to fully differentiated and/or genetically engineered cells. Cells can be induced to initiate clustering while in monolayer or in suspension.

The *in vitro* method of the current invention further includes steps to control optimal cluster size and to prevent further aggregation that may occur between pre-formed clusters. The method may further include steps for manipulation of the cells comprising a cluster prior, during or after cluster formation.

In one aspect, the invention provides *an in vitro* method for formation of cell clusters comprising:
a) selecting a group of cells to be clustered, wherein the cells are pancreatic, amniotic fluid derived or a co-culture of pancreatic and amniotic fluid derived cells;
b) expanding the cells in a monolayer;
c) forming a suspension of the cells in a liquid medium containing serum at a concentration of about 2% to about 10%;
d) incubating the medium containing the cells in a volumetric space bound by a surface, and;
e) limiting cellular attachment to the surface;
wherein the surface has a surface area and the step of limiting cellular attachment to the surface comprises limiting the surface area available for cellular attachment.

In another aspect, the invention provides *an in vitro* method for formation of cell clusters comprising:
a. selecting a group of cells to be clustered, wherein the cells are pancreatic, amniotic fluid derived or a co-culture of pancreatic and amniotic fluid derived cells;
b. expanding the cells in a monolayer;
c. forming a suspension of a subgroup of the cells in a liquid medium containing serum at a concentration of about 2% to about 10%;
d. introducing the medium containing the sub-group of cells in a volumetric space bounded by a surface;
e. allowing the sub-group of cells to adhere to the surface;
f. removing the cells of the sub-group of cells that have not adhered to the surface;
g. introducing the remainder of the cells; and
h. allowing the remainder of the cells to adhere to the cells adhering to the surface;
wherein the surface has a surface area and wherein the step of allowing the sub-group of cells to adhere to the surface comprises the step of limiting the surface area available for cellular adherence.

Cluster formation according to this method involves steps to limit or completely eliminate cell attachment to a surface by modifying that surface. So limiting attachment may be done by modifying a cell culture medium to further augment or reduce cell attachment. Alternatively, it can be done by introducing cells to a treated surface that has been modified to allow cell attachment only on selected spots on that surface.

In yet another embodiment, applying certain factors to the culture medium in a time dependent manner that may not at first but ultimately will limit or prevent the attachment of cells to the surface and, thereby, induce cluster formation. For example, factors that augment cell attachment may be first applied followed sequentially by other factors that reduce cell attachment to prevent further aggregation between formed clusters.

This invention also provides *an in vitro* method for controlling the size of a cluster. In one embodiment, the concentration of serum in a culture medium is adjusted to control the size of the cluster.

In another embodiment, the initial cell seeding density is a feature utilized to control the size of a cluster.

This invention also comprises an *in vitro* method for using a cell or a group of cells or a cell cluster that comprises certain characteristics to modify other cells that may be added to the cluster.

### BRIEF DESCRIPTION OF THE DRAWINGS

**Figure 1** depicts the typical morphology of pancreatic-derived cells grown in a monolayer culture.
**Figure 2** shows the morphology of amniotic fluid derived cells grown in a monolayer culture.
**Figure 3** shows clusters of pancreatic-derived cells formed in a suspension culture at a serum level of 2%.
**Figure 4** shows clusters of pancreatic-derived cells formed in a suspension culture at a serum level of 10%.
**Figure 5** shows clusters of pancreatic-derived cells formed in a suspension culture comprising 0.5E6 cells at a serum level of 2%
**Figure 6** shows clusters of pancreatic-derived cells formed in a suspension culture comprising 1.0E6 cells at a serum level of 2%.
**Figure 7** shows a cluster of cells growing outward from an edge that was created by a sterile scalpel on a low attachment hydrogel coated plate.
**Figure 8** shows a co-cluster comprised of pancreatic derived cells in red and amniotic derived cells in green formed in a suspension culture comprising both cell types at a 1:1 ratio.
**Figure 9** shows cells attached to a collagen IV coated island at 12 hours after incubation.
**Figure 10** shows cluster formation by cells attached to a collagen IV coated island at 24 hours after incubation.
**Figure 11** shows a floating cluster formed after 48 hours of initial cell attachment to a collagen IV coated island.
**Figure 12** shows real-time PCR data showing the expression level of the selected genes in pancreatic-derived cells grown in a monolayer (white bars) and pancreatic-derived cells grown in a cluster (black bars). Data shown are normalized to expression levels observed in adult human pancreas.

### DETAILED DESCRIPTION

The current invention comprises *an in vitro* method for inducing clustering of cells. A cell cluster may be thought of as a three-dimensional agglomeration of more than one cell where such cells are not formed in a monolayer. The cluster can be any shape, such as, for example, spheroid, ellipsoid, cuboid, and the like. Further, they may be non-uniform, non-asymmetrical agglomerations that form in three dimensions around a central cell or a small central group of cells.

A monolayer may be thought of as a single continuous layer of cells that is one cell or, at most, two cells in thickness. Cells in a monolayer may form groups or colonies of more than one cell, but these colonies are typically one cell in thickness.

The cells of the current invention may be either pancreatic, amniotic fluid derived or a co-culture of pancreatic and amniotic fluid derived cells. The cells may be autologous, allogeneic, or xenogeneic cells relative to each other and/or relative to an *in vivo* host for implantation purposes. The cells may be primary cells utilized directly after isolation from a donor. They are expanded in a monolayer prior to utilization. The cells may be undifferentiated or partially to fully differentiated and/or genetically engineered cells.

A cluster of the current invention may include starting materials comprised of a homogeneous population of cells of one type or a heterogeneous population of several types of cells. The ratio of different cell types contributing to a single cluster may be varied to optimize certain characteristics or to suppress certain characteristics of the cluster as a whole. The starting materials may be single cells or clusters of cells or combinations thereof. All of the cells contributing to a cluster may be introduced at once during the cluster induction process. Alternatively, additional cells may be introduced after initial cell seeding and cluster formation. Furthermore, additional clusters may be introduced after a starting cluster is formed whereby cluster aggregation or fusion may lead to the formation of composite clusters.

In one embodiment, a cell cluster may comprise a genetically modified cell that has a high expression of a specific gene or set of genes of interest. This high expression may then induce an up-regulation of genes of interest in other cell types within the cluster. Also, up regulated gene expression for a protein of interest in one cell type may induce a desired effect on responsive genes and signaling pathways of neighboring cells within a cluster.

In yet another embodiment, a cell cluster may comprise a xenogeneic cell of a specific phenotype that may have inherent surface ligands or receptors, secreted growth factors or cytokines able to influence neighboring cells within the cluster or in the immediate vicinity after implantation. Such effects may be mediated in part or in full by reconstitution of normal binding and signaling pathways between similar molecular entities of different species. The xenogeneic cell may be left in place or selectively removed prior to implantation by using unique cytotoxic antibodies and complement. Alternatively, if the xenogeneic cell (or the opposing cell(s) in the cluster) has been genetically engineered to be responsive to a unique selection agent, that agent may be added to the culture medium for a sufficient time to cause a cytotoxic effect followed by washout of the selection agent prior to implantation.

Survival of cells in a cluster is a function of the diffusion distance that nutrients and oxygen must travel to reach the innermost cells within a cluster. The optimum size of clusters of the current invention will vary depending on the application, but it must account for the diffusion distance necessary to maintain survival of the constituent cell types. It is clear to people with ordinary skills in the art that this distance will vary from one cell type to another. In one embodiment, this distance may be in the range of 2-1000 microns. Alternatively, it may be in the range of about 10 to about 500 microns. Alternatively, it may be in the range of about 10 to about 300 microns.

The total number of cells contributing to a cluster may also vary. A cluster may comprise 10-10,000 cells. Alternatively, it may comprise about 100 to about 10,000 cells. Alternatively, it may comprise about 1,000 to about 5,000 cells.

In one embodiment, a cell cluster may comprise cells involved in the secretion of insulin and the maintenance of glucose at a certain level, mimicking the function of islets of Langerhans that are found in the pancreas.

In another embodiment, a cell cluster may comprise cells involved in the construction of new blood vasculature such as endothelial cells. The presence of blood vessels may enhance cell survival within the body of a cluster after in vivo transplantation. Newly formed blood vessels may allow blood to penetrate farther into the body of a cluster, thereby reducing the overall diffusion distance necessary for efficient exchange of nutrients, gases, and waste.

According to the method of this invention, the induction of cell clustering may be achieved via several techniques. In one embodiment, cells that were grown in a monolayer are suspended in a medium appropriate for maintaining cell survival. The suspension of cells is then deposited in a vessel whose surface has been treated in a manner that inhibits or prevents cell attachment forming a system where the affinity of the cells for adherence to the vessel surface is lower than the affinity of cells to adhere to each other, leading initially to the formation of loosely adhered cell clusters. Shortly after, newly adhered cells produce endogenous extracellular matrix, thereby drawing the cells comprising the cluster into a more compact and stable structure.

Modifying a surface to prevent cell attachment may be achieved by a variety of techniques know to those who are experts in the art. For example, a surface may be coated with a hydrogel material that increases hydrophilicity and in turn decreases the affinity of cell membranes to adhere to the surface.

Cellular attachment is controlled by limiting the surface area available for normal attachment. A cell suspension is then introduced to the surface available for attachment. After the entire surface available for attachment is occupied, cells remaining in suspension are removed and those already attached are maintained and allowed to proliferate outward forming islands that eventually abandon the surface and form clusters.

In one embodiment, minimizing the surface available for attachment may occur by generating islands for cell attachment on a surface that otherwise prevents cell attachment. These islands are herein referred to as micro-spots. Micro-spots may be created by depositing droplets of a solution of an extracellular matrix protein on the surface. The surface is then allowed to dry. Alternatively, soft lithography could be utilized to create ECM micro-patterns on a surface that does not otherwise permit cell attachment. Each micro-spot could lead to the formation of at least one cluster. Persons of ordinary skill in the art will appreciate that there are many ways to modify a given surface to create micro-spots that allow cell attachment.

In another embodiment, a cell suspension could be introduced to a surface that does not otherwise permit cell attachment using micro-spots on the surface that allow such attachment. After the cells fill the space available for attachment, they may be removed, and a new cell type may be introduced in suspension, having an affinity of attachment to the first cell type, which is in turn attached to the micro-spots. This technique can be used to build a 3-D cluster comprised of various layers of cell types that mimic an *in vivo* structure.

In one embodiment, a surface that allows cell attachment may be modified by coating it with a material that does not allow cell attachment and then removing portions of the coating material, thereby re-exposing the surface underneath for cell attachment. **Example 4** is a representation of this technique where surgical blade was used to remove the hydrogel coating that prevents cell attachment, thereby exposing the original polystyrene surface, which subsequently accommodated cell attachment.

Cell density may influence cluster induction. Cell density herein refers to the number of cells per volume of medium. The higher the cell density is, the higher the probability of clustering and the faster the rate of cluster formation. In contrast, increasing the cell density beyond an optimal threshold may affect the ultimate survival of the cells and reduce the overall efficiency of clustering. This is clearly shown in **Example 2** where doubling the cell density from 0.5 x 10⁶ to 1.0 x 10⁶ cells per 6ml of culture medium led to a no significant increase in cluster formation. Those with ordinary skill in the art will appreciate the balance that is required by this invention between the speed of induction of clustering and the over all efficiency of the process demonstrated by the viability of the cells contributing to the clustering.

Another parameter affecting cluster formation is the concentration of serum in the culture medium. Serum concentration may vary according to the optimal cluster size required. The size of the clusters directly correlates with the serum concentration, again as shown in **Example 2.** The size of the clusters formed from pancreatic derived cells increased dramatically when the serum concentration was increased from 2% to 10%. In contrast, absence of serum from the culture medium may reduce clustering efficiency and lead to cell death.

Another technique for lowering the rate of cluster formation and limiting the size of a cluster relies on the timed introduction of factors that reduces the tendency of cells to attach to each other. Addition of these factors to the culture medium once cell clustering has occurred and the optimal cluster size has been achieved will reduce further attachment of single cells to formed clusters and also reduces aggregation between formed clusters.

### Example 1

### Preparation of pancreatic derived cells

*Pancreas Preparation -* Human pancreata not suitable for clinical transplantation were obtained from The National Disease Research Interchange (Philadelphia, PA), following appropriate consent for research use. The pancreas was transferred with organ preservation solution to a stainless steel pan on ice and trimmed of all extraneous tissue. The pancreatic duct was cannulated with an 18 gauge catheter and the pancreas was injected with an enzyme solution containing LIBERASE HI ™ enzyme (Roche - 0.5 mg/ml) and DNase I (0.2 mg/ml) dissolved in Dulbecco's Phosphate Buffered Saline (DPBS).

*Gradual Mechanical Dissociation with Simultaneous Enzyme Digestion -* The enzyme infused pancreata were processed according to methods as described in Diabetes 37:413-420 (1988). Briefly, the pancreata were cleaned of extraneous tissue and injected with the enzyme solution as described above. The pancreata were then placed into a Ricordi Chamber with stainless steel beads and covered with a screen having a mesh size of 400-600 µm to retain larger pieces of tissue. The chamber was covered and the enzyme solution was circulated through the chamber at approximately 37°C, and the chamber was shaken to allow the beads to disrupt pancreatic tissue while the enzyme digested the pancreas. Once adequate dissociation and digestion was achieved, the digestion was terminated and the tissue was collected.

*Tissue Separation -* The collected tissue was centrifuged at 150 x g for 5 minutes at 4°C. The supernatant was aspirated, and the tissue was washed two additional times in DPBS. Following the final wash, the tissue was applied to a discontinuous gradient for purification. The digested tissue was suspended in polysucrose (Mediatech, VA) with a density of 1.108 g/ml at a ratio of about 1 to about 2 ml tissue pellet per 10 ml of polysucrose solution. The tissue suspension was then transferred to round-bottom polycarbonate centrifuge tubes and polysucrose solutions with densities of 1.096 and 1.037, respectively were carefully layered over the tissue suspension in the tubes. A final layer of DMEM completed the discontinuous purification gradient. The gradient tubes were centrifuged at 2000 rpm for 20 minutes at 4°C with no brake applied. Following centrifugation, the tissue was individually collected from each interface fraction (three interfaces), washed several times in DPBS+ as described above, and collected in a 50 ml test tube.

*Further Cell Cluster Dissociation -* Optionally, one can further dissociate large cell clusters obtained using the above protocol into smaller clusters or single cell suspensions. After the final wash, the tissue from each fraction was suspended in 10 ml 1x trypsin/EDTA solution containing 200U/mL DNase I. The tubes were placed in the water bath and repeatedly aspirated and discharged from a 10 ml serological pipette for 5 to 6 minutes until a near single cell suspension was achieved. The digestion was quenched with the addition of 4°C DPBS+ and the tubes centrifuged at 800 rpm for 5 minutes. The cell suspensions were washed with DPBS+ and cultured as described below.

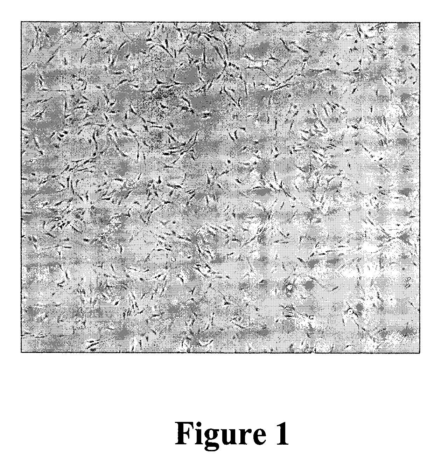

*Pancreatic Cell Culture -* Following the final wash, the cells from each interface were resuspended in DMEM, 2% FBS, 100 U/µg penicillin/streptomycin, ITS, 2 mM L-Glutamine, 0.0165 mM ZnSO₄ (Sigma), and 0.38 µM 2-mercaptoethanol (Invitrogen, CA) (hereinafter "the selection media"). Aliquots of six ml of the cell suspension were seeded in T-25 tissue culture flasks or alternatively 12 ml of the cell suspension was seeded into T-75 flasks. The flasks were placed in 37°C incubators with 5% CO₂. Following 2 to 4 weeks culture, a complete medium change was performed, and adherent cells were returned to culture in DMEM (2750 mg/L D-glucose, 862 mg/L glutamine) (Gibco, CA) with 5% FBS (HyClone, UT), 1% P/S, 0.0165 mM ZnSO₄ (hereinafter "the growth medium") and allowed to reach near confluence (this stage is referred to as "passage 0" or "P0"), at which point they were passaged. Subsequent culturing of the cells was at 5000 cell/cm² in the growth medium. Cultures were passaged every 7 to 10 days after reaching approximately 70-90% confluency. **Figure 1** depicts the typical stromal morphology of pancreatic-derived cells.

### Example 2

### Clustering of Pancreatic Cells

Pancreatic cells isolated according to **Example 1** were cultured in suspension in low attachment 6-well plates (Corning Life Sciences). The media used comprised DMEM-low glucose supplemented with 0, 2, or 10% FBS. Wells contained 0.25 x 10⁶, 0.5 x 10⁶, or 1.0 x 10⁶ cells. Cells were cultured in suspension for two days. Clustering efficiency was evaluated based on cluster size, viability, uniformity, and number.

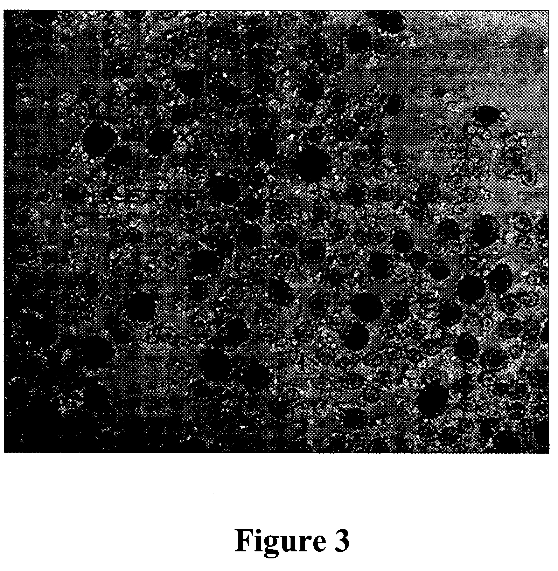

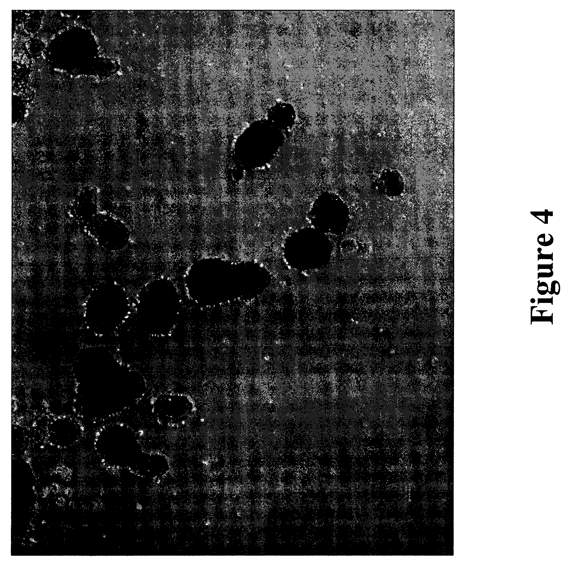

Visual analysis under a microscope clearly showed that cell density and serum concentration play a key role in shaping the geometry of the clusters. Serum enhances the ability of the cells to attach to each other and also the ability of the clusters to aggregate and increase in size. For a seeding density of 0.5 x 10⁶ cells, increasing the medium FBS level from 2% (**Figure 3**) to 10% (**Figure 4**) led to a large increase in cluster size and in turn led to central necrosis of the resulting clusters, as indicated by the dark cluster centers.

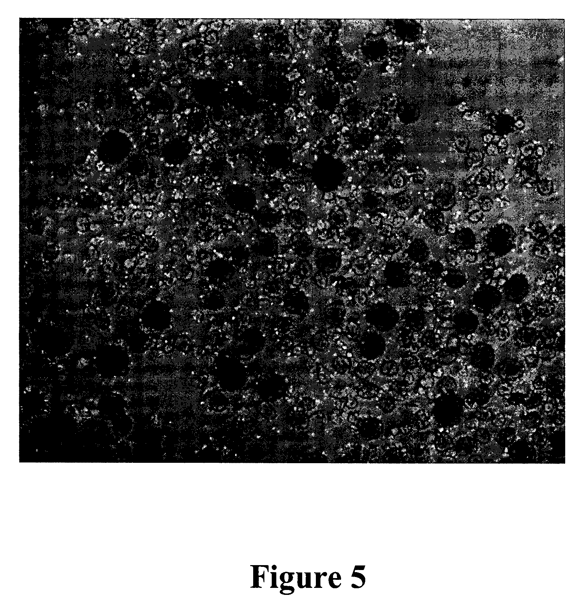

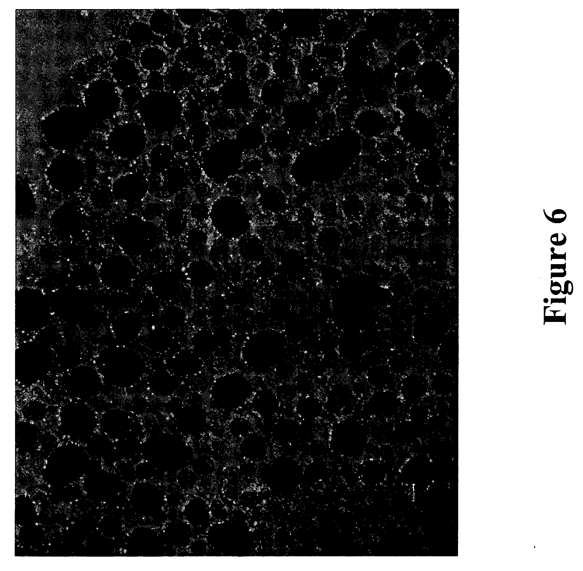

Visual analysis also revealed that when the concentration of serum was kept constant at 2% and the seeding number of cells was varied between 0.5 x 10⁶ (**Figure 5**) and 1.0 x 10⁶ (**Figure 6**), the overall number of clusters did not vary significantly. The lower seeding cell number favored optimal cluster size and retention of cell viability, as shown by comparison of the two figures.

### Example 3

### Viability Testing

Clusters were formed according to **Example 1** with 0.5 x 10⁶ cells per well in a medium containing DMEM-LG supplemented with 2% FBS. On day three, clusters were removed from the well and pipetted into a 50ml tube (Falcon). The well was then washed with PBS and pooled with the contents of the tube to ensure collection of all clusters. Clusters were then centrifuged at 200 rpm for 2 minutes, the supernatant was removed and replaced with 5ml of TRYPLE™ Express (Invitrogen, CA), then incubated for 10 minutes. Pipetting several times helped further to break apart the clusters. The cell suspension was then evaluated under the microscope to ensure preparation of a single cell suspension. Cell viability was evaluated using a Guava PCA-96 cell analysis system and the VIACOUNT® reagent (Guava, CA). The count obtained was an average of three separate trials. The total cell viability was measured to be 76%, demonstrating that the majority of cells comprising the clusters were viable cells.

### Example 4

### Alternative clustering technique

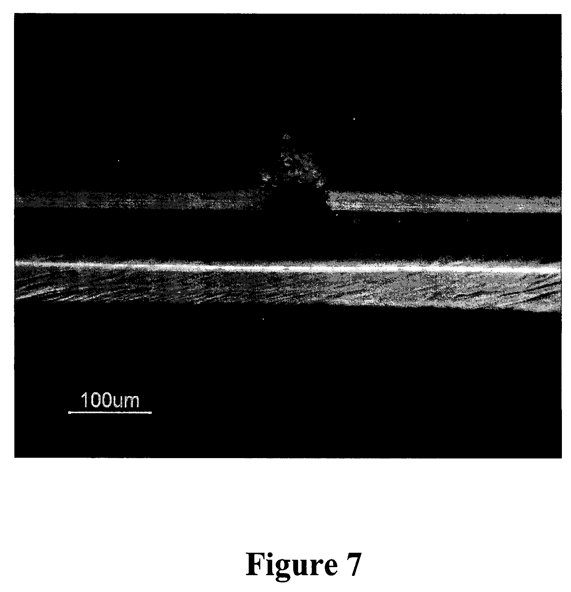

Edges were created on the surface of a 10cm low attaching plate using a sterile scalpel to remove the low attachment hydrogel coating. This process exposed the polystyrene surface underneath. A 5ml solution of 10µg/ml collagen type IV (BD Biosciences) in PBS was then pipetted into the plate and allowed to incubate for an hour. Collagen bound only along edges where the low attachment hydrogel had been removed. The collagen solution was then aspirated to remove, and a single cell suspension of 1.0 x 10⁶ cells in DMEM-LG with 2% FBS was pipetted into the plate. Cells were allowed to incubate overnight in an atmosphere of 37°C with 5% CO₂. Non-adherent cells were then removed and fresh medium was added. A complete medium change was conducted on alternating days. At day 10, attached clusters appeared at the exposed edges, eventually becoming non-adherent clusters **(****Figure 7****).**

### Example 5

### Amniotic derived cells

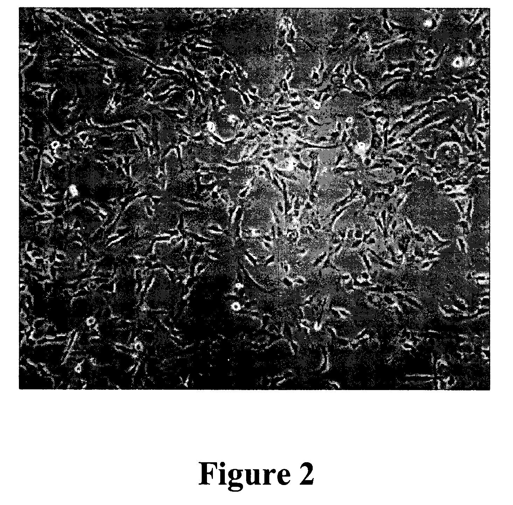

Amniotic fluid used to isolate cells of the present invention was taken from specimens obtained from routine amniocentesis performed at 17 to 22 weeks gestation for routine fetal karyotyping. The amniotic fluid was centrifuged for 7 minutes at 400-x g and the supernatant removed. The resulting cell pellet was re-suspended in Amniomax® growth medium (Gibco, MD, USA). The cells were cultured on fibronectin (10 µg/ml) (BD Biosciences, CA, USA) coated plates. The cultures were left undisturbed for at least 5 to 10 days under hypoxic conditions (3% O₂) after which the cultures were fed with the same growth medium and cultured until reaching approximately 70 to 80% confluency. Cells at this stage were referred to as "P0". The amniotic fluid derived (AF) cells used for purposes of this invention were at expansion passage 10. **Figure 2** depicts typical amniotic fluid derived cell morphology.

### Example 6

### Clustering of Amniotic Fluid Derived cells

Two lines of AF cells were used for this experiment. The lines were established as indicated in the above example. Clustering was induced using the method outlined above in example 2. Cells were cultured in low attachment 6-well plates in DMEM-LG supplemented with 2% FBS. Clusters were evaluated at 48hrs. One of the two lines (Line A) formed clusters. The second cell line (Line B) did not respond to the cluster inducing method, even when the culture time was extended to 1 week.

### Example 7

### Co-culturing as a method for inducing cluster formation

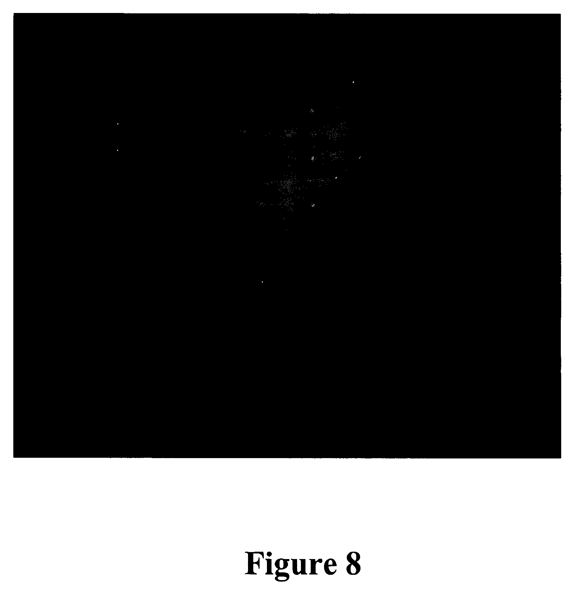

This experiment was carried out using two cell types: pancreatic derived cells and AF cells line B as derived in examples above. Clustering was induced using the method outlined above in **Example 2.** Cells were cultured in low attachment 6-well plates in DMEM-LG supplemented with 2% FBS. Clusters were evaluated at 48hrs. Each well was comprised of a starting density of 0.5x 10⁶ cells of either pancreatic derived cells alone, AF line B cells alone, or a 1:1 ratio of the two cell types mixed together. As expected, pancreatic derived cells cultured alone formed clusters within 48hrs. AF line B cells did not form any clusters even when the culture was extended to 1 week. In contrast, the combination of the two cell types formed clusters within 48hrs. To insure that the clusters comprised both cell types, each cell type was tagged with a different fluorescent marker using a vibrant multi-color cell labeling kit (Molecular Probes, OR, USA). Following cluster induction, the clusters were analyzed under a fluorescent microscope. **Figure 8** shows a cluster comprising both cell types.

### Example 8

### Micro-islands as a method for inducing clustering

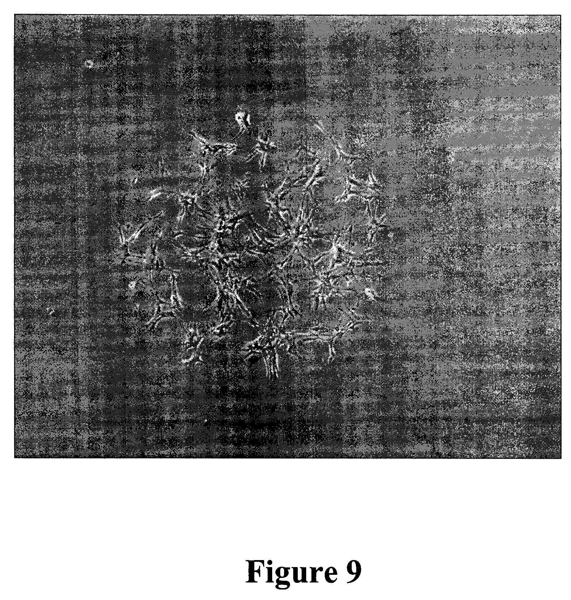

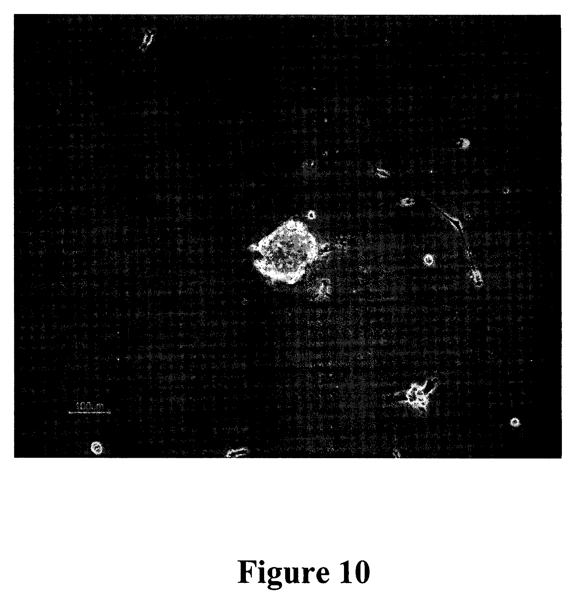

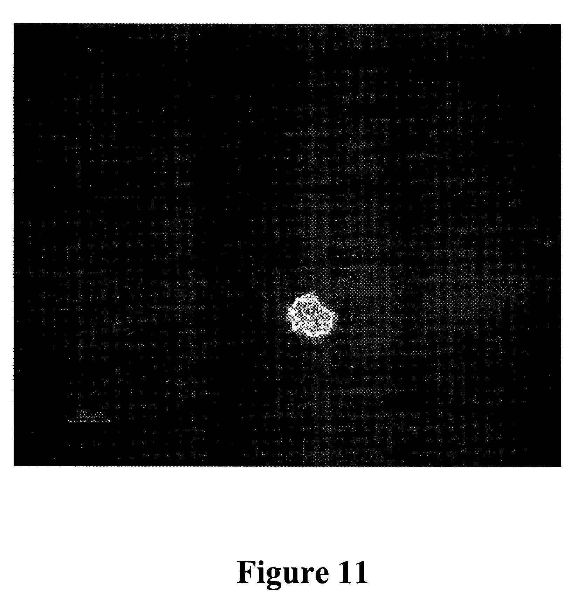

Pancreatic derived cells were obtained via methods outlined in **Example 2.** Cells were cultured and maintained in DMEM-LG medium supplemented with 10% FBS. A collagen IV (Vendor??) solution was prepared in PBS at a concentration of 20µg/ml. Hydrogel coated (diameter = 10 cm) (Corning, NY) low attachment plates were used for this experiment. 0.21µl droplets of the collagen IV solution were deposited on the plate surface, and the plate was incubated at room temperature for 1 hour. A 10ml cell suspension of pancreatic derived cells in DMEM-LG medium supplemented with 10% FBS was pipetted slowly onto the surface of the plate. The plate was incubated for 3 hours at 37°C in an atmosphere of 5%CO₂. The following day, a complete medium change was conducted to remove all cells remaining in suspension. Cells attached to the collagen IV coated surface formed a pattern of islands (**Figure 9**). At 24 hours, cells had spread to fill the intervening spaces between the islands (**Figure 10**). At 48 hrs cells gathered into clusters (**Figure 11**) and eventually detached from the surface of the plate.

### Example 9

### PCR Analysis for pancreatic derived cells in monolayer and in clusters

Pancreatic derived cells isolated according to **Example 1** were either cultured in monolayer or induced to cluster according to the method described in example 2.

RNA was extracted from either single cells grown in monolayer or clusters of cells. Total RNA from human pancreas (Ambion, INC.) was used as positive control.

*RNA extraction, purification, and cDNA synthesis:* RNA samples were purified through its binding to a silica-gel membrane (Rneasy Mini Kit, Qiagen, CA) in the presence of an ethanol-containing, high-salt buffer; while contaminants were washed away. The RNA was further purified while bound to the column by treatment with DNase I (Qiagen, CA) for 15 min. High-quality RNA was then eluted in water. Yield and purity were assessed by A260 and A280 readings on the spectrophotometer. cDNA copies were made from purified RNA using an ABI (ABI, CA) high capacity cDNA archive kit.

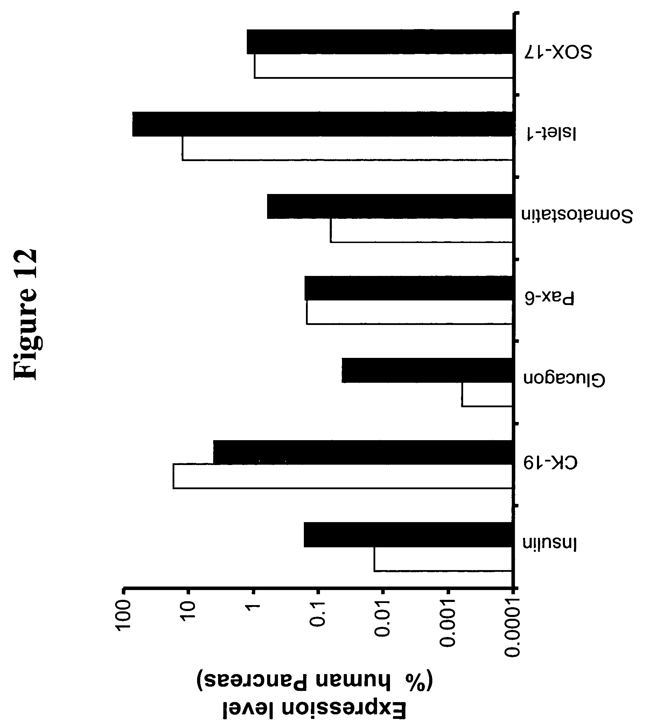

*Real-time PCR amplification and quantitative analysis:* Unless otherwise stated, all reagents were purchased from Applied Biosystems. Real-time PCR reactions were performed using the ABI PRISM® 7000 Sequence Detection System. TAQMAN® UNIVERSAL PCR MASTER MIX® (ABI, CA) was used with 20 ng of reverse transcribed RNA in a total reaction volume of 20 µl. Each cDNA sample was run in duplicate to correct for pipetting errors. Primers and FAM-labeled TAQMAN®probes were used at concentrations of 200 nM. The level of expression of each target gene was normalized using the pre-developed Applied Biosystem's 18S ribosomal RNA or human glyceraldehydes-3-phosphate dehydrogenase (GAPDH) endogenous control kit. Primers and probes were either designed using ABI PRISM PRIMER EXPRESS™ software or used pre-developed ABI gene analysis kit. For each gene, either one of the primers or the probe were designed to be exon-boundary spanning. This eliminated the possibility of the primers/probe binding to any genomic DNA present. The primer and probe sets are listed as following Pax6 (Hs00240871), Insulin (Hs00355773), Glucagon (Hs00174967), Isl-1 (Hs00158126), Somatostatin (Hs00174949), The remaining primers were designed by using the PRIMERS program (ABI, CA). After an initial 50°C for 2 min, and 95°C for 10 min, samples were cycled 40 times in two stages - a denaturation step at 95°C for 15 sec, followed by an annealing/extension step at 60°C for 1 min. Data analysis was carried out using GENEAMP®7000 Sequence Detection System software. For each primer/probe set, a Cₜ value was determined as the cycle number at which the fluorescence intensity reached a specific value in the middle of the exponential region of amplification. Relative gene expression levels were calculated using the comparative Cₜ method. Briefly, for each cDNA sample, the endogenous control Cₜ value was subtracted from the gene of interest Cₜ to give the delta Cₜ value (ΔCₜ). The normalized amount of target was calculated as 2^{-ΔCt}, assuming amplification to be 100% efficiency. Final data were expressed relative to a calibrator sample. The comparative Cₜ method is only valid if target and endogenous control amplification efficiencies are approximately equal. Preliminary validation experiments were therefore performed for each primer/probe set by amplifying serially diluted cDNA samples and determining the ΔCₜ values. These ΔCₜ values should remain constant across the range of dilutions if amplification efficiencies are equal. **Figure 12** shows a comparison between the gene expression levels for cells in monolayer verses those in clusters.

## Claims

1. An *in vitro* method for formation of cell clusters comprising:
a. selecting a group of cells to be clustered, wherein the cells are pancreatic, amniotic fluid derived or a co-culture of pancreatic and amniotic fluid derived cells;
b. expanding the cells in a monolayer;
c. forming a suspension of the cells in a liquid medium containing serum at a concentration of about 2% to about 10%;
d. incubating the medium containing the cells in a volumetric space bounded by a surface; and
e. limiting cellular attachment to the surface;
wherein the surface has a surface area and the step of limiting cellular attachment to the surface comprises limiting the surface area available for cellular attachment.

2. The method of claim 1, wherein the surface has been modified to allow cell attachment only on selected spots of the surface.

3. The method of claim 1, wherein limiting the surface area comprises coating the surface with a material that inhibits cellular attachment.

4. The method of claim 3 wherein the material that inhibits cellular attachment prevents cellular attachment.

5. The method of claim 4 wherein portions of the material that inhibits cellular attachment are removed.

6. The method of claim 1 wherein the step of limiting cellular attachment to the surface comprises lowering the binding affinity of the surface for the cells to a value less than the binding affinity of the cells to themselves.

7. The method of claim 1, wherein the clusters form on the surface.

8. The method of claim 1 wherein the group of cells to be clustered comprise 2 or more cell types.

9. The method of claim 1 further comprising depositing extracellular matrix protein on areas of said surface.

10. An *in vitro* method for formation of cell clusters comprising:
a. selecting a group of cells to be clustered, wherein the cells are pancreatic, amniotic fluid derived or a co-culture of pancreatic and amniotic fluid derived cells;
b. expanding the cells in a monolayer;
c. forming a suspension of a subgroup of the cells in a liquid medium containing serum at a concentration of about 2% to about 10%;
d. introducing the medium containing the sub-group of cells in a volumetric space bounded by a surface;
e. allowing the sub-group of cells to adhere to the surface;
f. removing the cells of the sub-group of cells that have not adhered to the surface;
g. introducing the remainder of the cells; and
h. allowing the remainder of the cells to adhere to the cells adhering to the surface;
wherein the surface has a surface area and wherein the step of allowing the sub-group of cells to adhere to the surface comprises the step of limiting the surface area available for cellular adherence.

11. The method of claim 10 wherein limiting the surface area comprises coating the surface with a material that inhibits cellular attachment.

12. The method of claim 10 wherein the material that inhibits cellular attachment prevents cellular attachment.

13. The method of claim 11 wherein portions of the material that inhibits cellular attachment are removed.

14. The method of claim 10 wherein cellular adherence is limited by lowering the binding affinity of the surface for the cells to a value less than the binding affinity of the cells to themselves.

15. The method according to claim 11, wherein the clusters form on the surface.

16. The method of claim 10, wherein the group of cells to be clustered comprise 2 or more cell types.

17. The method of claim 10, further comprising depositing extracellular matrix protein on limited areas of said surface.

18. The method of claim 1 or claim 10, wherein the liquid medium contains serum at a concentration of about 2%.

## Patentansprüche

1. *In*-*vitro*-Verfahren zur Bildung von Zell-Clustern, das Folgendes umfasst:
a. Auswählen einer Gruppe von Zellen, aus denen Cluster gebildet werden sollen, wobei es sich bei den Zellen um Pankreaszellen, von Amnionflüssigkeit abgeleitete Zellen oder eine Cokultur aus Pankreaszellen und aus Amnionflüssigkeit abgeleiteten Zellen handelt;
b. Expandieren der Zellen zu einer Einzelzellschicht;
c. Bilden einer Suspension der Zellen in einem Flüssigmedium, das Serum in einer Konzentration von ungefähr 2% bis ungefähr 10% enthält;
d. Inkubieren des Mediums, das die Zellen enthält, in einem von einer Oberfläche begrenzten Volumensraum; und
e. Einschränken des Verankerns der Zellen an der Oberfläche;
wobei die Oberfläche über eine bestimmte Oberfläche verfügt und der Schritt des Einschränkens des Verankerns der Zellen an der Oberfläche das Einschränken der bestimmten Oberfläche, die für das Verankern der Zellen verfügbar ist, umfasst.

2. Verfahren nach Anspruch 1, wobei die Oberfläche so modifiziert worden ist, dass sie ein Verankern der Zellen nur an ausgewählten Stellen der Oberfläche gestattet.

3. Verfahren nach Anspruch 1, wobei das Einschränken der bestimmten Oberfläche das Beschichten der Oberfläche mit einem Material, das das Verankern der Zellen hemmt, umfasst.

4. Verfahren nach Anspruch 3, wobei das Material, das das Verankern der Zellen hemmt, das Verankern der Zellen verhindert.

5. Verfahren nach Anspruch 4, wobei Teile des Materials, das das Verankern der Zellen hemmt, entfernt werden.

6. Verfahren nach Anspruch 1, wobei der Schritt des Einschränkens des Verankerns der Zellen an der Oberfläche das Vermindern der Bindungsaffinität der Oberfläche für die Zellen auf einen Wert von weniger als die Bindungsaffinität der Zellen aneinander umfasst.

7. Verfahren nach Anspruch 1, wobei sich die Cluster an der Oberfläche bilden.

8. Verfahren nach Anspruch 1, wobei die Gruppe von Zellen, aus denen Cluster gebildet werden sollen, 2 oder mehr Zelltypen umfasst.

9. Verfahren nach Anspruch 1, das weiterhin das Ablegen eines extrazellulären Matrixproteins an Flächen der Oberfläche umfasst.

10. In-vitro-Verfahren zur Bildung von Zell-Clustern, das Folgendes umfasst:
a. Auswählen einer Gruppe von Zellen, aus denen Cluster gebildet werden sollen, wobei es sich bei den Zellen um Pankreaszellen, von Amnionflüssigkeit abgeleitete Zellen oder eine Cokultur aus Pankreaszellen und aus Amnionflüssigkeit abgeleiteten Zellen handelt;
b. Expandieren der Zellen zu einer Einzelzellschicht;
c. Bilden einer Suspension einer Untergruppe der Zellen in einem Flüssigmedium, das Serum in einer Konzentration von ungefähr 2% bis ungefähr 10% enthält;
d. Einführen des Mediums, das die Untergruppe von Zellen enthält, in einen von einer Oberfläche begrenzten Volumensraum;
e. Verankernlassen der Untergruppe von Zellen an der Oberfläche;
f. Entfernen der Zellen der Untergruppe von Zellen, die sich nicht an der Oberfläche verankert haben;
g. Einführen der restlichen Zellen; und
h. Verankernlassen der restlichen Zellen an die Zellen, die an der Oberfläche verankert sind;
wobei die Oberfläche über eine bestimmte Oberfläche verfügt und wobei der Schritt des Verankernlassens der Untergruppe von Zellen an der Oberfläche den Schritt des Einschränkens der für die Zellverankerung verfügbaren bestimmten Oberfläche umfasst.

11. Verfahren nach Anspruch 10, wobei das Einschränken der bestimmten Oberfläche das Beschichten der Oberfläche mit einem Material, das das Verankern der Zellen hemmt, umfasst.

12. Verfahren nach Anspruch 10, wobei das Material, das das Verankern der Zellen hemmt, das Verankern der Zellen verhindert.

13. Verfahren nach Anspruch 11, wobei Teile des Materials, das das Verankern der Zellen hemmt, entfernt werden.

14. Verfahren nach Anspruch 10, wobei die Zellverankerung durch das Vermindern der Bindungsaffinität der Oberfläche für die Zellen auf einen Wert von weniger als die Bindungsaffinität der Zellen aneinander eingeschränkt ist.

15. Verfahren nach Anspruch 11, wobei sich die Cluster an der Oberfläche bilden.

16. Verfahren nach Anspruch 10, wobei die Gruppe von Zellen, aus denen Cluster gebildet werden sollen, 2 oder mehr Zelltypen umfasst.

17. Verfahren nach Anspruch 10, das weiterhin das Ablegen eines extrazellulären Matrixproteins an eingeschränkten Flächen der Oberfläche umfasst.

18. Verfahren nach Anspruch 1 oder Anspruch 10, wobei das Flüssigmedium Serum in einer Konzentration von ungefähr 2% enthält.

## Revendications

1. Procédé *in vitro* de formation d'amas cellulaires, comprenant :
a. la sélection d'un groupe de cellules à regrouper, les cellules étant des cellules pancréatiques, des cellules qui dérivent du liquide amniotique, ou une co-culture de cellules pancréatiques et de cellules qui dérivent du liquide amniotique ;
b. l'expansion des cellules en une monocouche ;
c. la formation d'une suspension des cellules dans un milieu liquide contenant du sérum à une concentration d'environ 2 % à environ 10 % ;
d. l'incubation du milieu contenant les cellules dans un espace volumétrique délimité par une surface ; et
e. la limitation de l'attachement des cellules à la surface ;
dans lequel la surface a une aire, et l'étape de limitation de l'attachement des cellules à la surface comprend la limitation de l'aire disponible pour l'attachement des cellules.

2. Procédé selon la revendication 1, dans lequel la surface a été modifiée de façon à ne permettre l'attachement des cellules que sur des taches sélectionnées de la surface.

3. Procédé selon la revendication 1, dans lequel la limitation de l'aire comprend le revêtement de la surface par un matériau qui inhibe l'attachement des cellules.

4. Procédé selon la revendication 3, dans lequel le matériau qui inhibe l'attachement des cellules empêche l'attachement des cellules.

5. Procédé selon la revendication 4, dans lequel les portions du matériau qui inhibe l'attachement des cellules sont enlevées.

6. Procédé selon la revendication 1, dans lequel l'étape de limitation de l'attachement des cellules à la surface comprend l'abaissement de l'affinité de liaison de la surface vis-à-vis des cellules, à une valeur inférieure à l'affinité de liaison des cellules à elles-mêmes.

7. Procédé selon la revendication 1, dans lequel les amas se forment sur la surface.

8. Procédé selon la revendication 1, dans lequel le groupe de cellules à regrouper comprend deux types cellulaires, ou plus.

9. Procédé selon la revendication 1, qui comprend en outre le dépôt d'une protéine de la matrice extracellulaire sur des zones de ladite surface.

10. Procédé *in vitro* de formation d'amas cellulaires, comprenant :
a. la sélection d'un groupe de cellules à regrouper, les cellules étant des cellules pancréatiques, des cellules qui dérivent du liquide amniotique, ou une co-culture de cellules pancréatiques et de cellules qui dérivent du liquide amniotique ;
b. l'expansion des cellules en une monocouche ;
c. la formation d'une suspension d'un sous-groupe des cellules dans un milieu liquide contenant du sérum à une concentration d'environ 2 % à environ 10 % ;
d. l'introduction du milieu contenant le sous-groupe de cellules dans un espace volumétrique délimité par une surface ;
e. le fait de permettre au sous-groupe de cellules d'adhérer à la surface ;
f. l'enlèvement des cellules du sous-groupe de cellules qui n'ont pas adhéré à la surface ;
g. l'introduction du reste des cellules ; et
h. le fait de permettre au reste des cellules d'adhérer aux cellules adhérant à la surface ;
dans lequel la surface à une aire, et l'étape consistant à permettre au sous-groupe de cellules d'adhérer à la surface comprend l'étape de limitation de l'aire disponible pour l'adhérence des cellules.

11. Procédé selon la revendication 10, dans lequel la limitation de l'aire comprend le revêtement de la surface par un matériau qui inhibe l'attachement des cellules.

12. Procédé selon la revendication 10, dans lequel le matériau qui inhibe l'attachement des cellules empêche l'attachement des cellules.

13. Procédé selon la revendication 11, dans lequel les portions du matériau qui inhibe l'attachement des cellules sont enlevées.

14. Procédé selon la revendication 10, dans lequel l'adhérence des cellules est limitée par abaissement de l'affinité de liaison de la surface vis-à-vis des cellules à une valeur inférieure à l'affinité de liaison des cellules à elles-mêmes.

15. Procédé selon la revendication 11, dans lequel les amas se forment sur la surface.

16. Procédé selon la revendication 10, dans lequel le groupe de cellules à regrouper comprend deux types cellulaires, ou plus.

17. Procédé selon la revendication 10, qui comprend en outre le dépôt d'une protéine de la matrice extracellulaire sur des zones limitées de ladite surface.

18. Procédé selon la revendication 1 ou la revendication 10, dans lequel le milieu liquide contient du sérum à une concentration d'environ 2 %.
